Europäisches Patentamt

European Patent Office

Office européen des brevets

(11) Veröffentlichungsnummer: **0 296 469 B1**

(12) **EUROPÄISCHE PATENTSCHRIFT**

(45) Veröffentlichungstag der Patentschrift: **15.12.93**

(21) Anmeldenummer: **88109513.7**

(22) Anmeldetag: **15.06.88**

(51) Int. Cl.⁵: **G01N 33/36**, B65H 63/06

(54) **Verfahren und Vorrichtung zur Oualitätsbestimmung an laufenden Garnen.**

(30) Priorität: **24.06.87 DE 3720835**

(43) Veröffentlichungstag der Anmeldung:
**28.12.88 Patentblatt 88/52**

(45) Bekanntmachung des Hinweises auf die
Patenterteilung:
**15.12.93 Patentblatt 93/50**

(84) Benannte Vertragsstaaten:
**AT BE CH DE FR GB IT LI LU NL**

(56) Entgegenhaltungen:
**DE-A- 2 749 563      DE-A- 2 933 297
DE-B- 2 912 577      FR-A- 1 271 483
US-A- 3 729 635      US-A- 3 758 216**

(73) Patentinhaber: **HOECHST AKTIENGESELL-
SCHAFT**

**D-65926 Frankfurt(DE)**

(72) Erfinder: **Scholz, Klaus-Dieter
Wacholderstrasse 1
D-8903 Bobingen(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein Verfahren zur Bestimmung von Qualitätskenndaten laufender Garne mit Hilfe von Flusenzählern.

Die Überwachung laufender Garne auf Durchmesserschwankungen und besonders auf abstehende Filamente, d.h. Flusen oder Schlaufen, bei der Herstellung von Garnen in der Textil- oder Chemiefaserindustrie mit Hilfe strahlenoptischer Vorrichtungen, z.B. von Lichtschranken, ist bekannt.

Eine derartige Vorrichtung zum Überwachen eines einzelnen laufenden Fadens oder Garnes auf Durchmesserschwankungen und abstehende Filamente mit Hilfe einer im Infrarotgebiet arbeitenden Lichtschranke ist z.B. aus der DE-OS 29 33 297 bekannt.

Bei der Kettbaumherstellung werden mit ähnlichen Lichtschranken Scharen von einigen hundert ablaufenden Garnen überwacht. Dabei werden nur relativ grobe Garnfehler und abgerissenes Garn als Fehler erfaßt; gerissene Einzelfilamente an einzelnen Garnen können diese Lichtschranken nicht erkennen.

Für Prüfzwecke und zur Qualitätskontrolle werden ablaufende Kettfadenscharen in einem Prüfshärgatter bisher visuell überwacht und so bewertet.

Es ist naheliegend, derartige Prüfschärgatter mit je einer Lichtschranke oder je einem sonstigen sogenannten Flusenzähler pro laufendes Garn zu überwachen. Derartige Anordnungen liefern jedoch keine reproduzierbaren Qualitätskenndaten für ablaufende Kettgarnscharen eines Schärgatters. Es hat sich nämlich gezeigt, daß bei den herkömmlichen Meßverfahren in statistischer Folge Messergebnisse registriert werden, die weit außerhalb der Werte liegen, die der tatsächlichen Qualität des gemessenen Fadens entsprechen. Die Ursachen für solche Ausreißerergebnisse sind weitgehend unbekannt. Sie haben jedoch zur Folge, daß die Beurteilung der Qualität eines Fadens anhand derartiger Meßverfahren in der Praxis nicht möglich ist und daher nach wie vor eine visuelle Beurteilung des laufenden Fadens erforderlich ist.

Es ist auch bereits aus der DE-A-27 49 563 ein Verfahren zur Erkennung von periodischen Dickeschwankungen von Garnen bekannt, bei welchem das Garn mehrere Lichtschranken durchläuft, die in bestimmten Abständen hintereinander im Fadenlauf liegen. Man stellt bei diesem bekannten Verfahren den Lichtschrankenabstand so ein, daß der gemessene Garndicke-Verlauf an allen Lichtschranken in Phase ist. Ist dies der Fall, so ist der gesuchte Periodenabstand gleich oder ein ganzzahliges Vielfaches des Lichtschrankenabstands.

Diese aufwendige Vorrichtung eignet sich nur zum Erkennen periodisch auftretender Dickeschwankungen, nicht aber zu einer Bestimmung und Kennzeichnung der Qualität laufender Garne.

Überraschenderweise hat sich nun gezeigt, daß man auch durch eine automatische Flusenzählung zu einem die Qualität des Fadens gut wiederspiegelden Zahlenwert gelangen kann, der auch gut reproduzierbar ist, wenn erfindungsgemäß der Faden mindestens drei gleichartige, hintereinander angeordnete Flusensensoren durchläuft, deren bei Durchlauf eines Garnabschnitts der frei gewählten Länge L abgegebenen Zählimpulse in einen längenbezogenen Mittelwert R umgerechnet werden, wobei weit abweichende Einzelmeßergebnisse als Ausreißerwerte nicht in die Mittelwertbildung einbezogen werden.

Da das Eliminieren von Einzel-Meßergebnissen aus einer Meßreihe normalerweise zu einem Mittelwert von verringerter Verläßlichkeit führt, war es nicht zu erwarten, daß sich hier durch diese Maßnahme eine gut reproduzierbare Meßgröße für den Faden ergibt, die seine Qualität weit besser kennzeichnet als ein aus einer weit höheren Zahl von Einzelmessungen in üblicher Weise errechneter Mittelwert.

Das erfindungsgemäße Verfahren wird im einzelnen so ausgeführt, daß man den zu bewertenden Faden nacheinander mindestens drei gleichartige Flusensensoren ($S_1$, $S_2$, $S_3$... $S_n$) durchlaufen läßt. Die während des Durchlaufs eines Fadenabschnitts der frei wählbaren, nicht zu kurzen Länge L von den einzelnen Sensoren abgegebenen Zählimpulse werden für jeden der Flusensensoren $S_1$ bis $S_n$ getrennt zu den Impulssummen $X_1$ bis $X_n$ addiert und gespeichert. Die Indices i (1 bis n) ermöglichen die Zuordnung der Impulssummen X zu den einzelnen Sensoren, indem man die Impulssumme mit dem gleichen Index versieht, wie den entsprechenden Sensor. Die Indices i durchlaufen somit die Werte der ganzen Zahlen von 1 bis n.

Finden sich in dem erhaltenen Wertekollektiv $X_1$ bis $X_n$ ein oder mehrere Werte $X_j^*$, die sehr stark von den übrigen Werten $X_i$ abweichen, sogenannte Ausreißerwerte, so werden diese Werte bei der Mittelwertbildung nicht berücksichtigt.

Als Mittelwert gilt dann bei N Ausreißerwerten:

$$\overline{X}^* = \frac{\sum\limits_{i=1}^{i=n} X_i - \sum\limits_{j=1}^{j=N} X_j^*}{n-N} \qquad (I)$$

Dieser korrigierte Mittelwert $\overline{X}_{korr}$ kann auch dadurch bestimmt werden, daß die Ausreißerwerte erst gar nicht in die Mittelwertbildung einbezogen werden.

Unabhängig von der Rechenstrategie erhält man den durch die Formel I definierten Wert von $\overline{X}_{korr}$.

$\overline{X}_{korr}$ ergibt nach Normierung auf eine Längeneinheit der gemessenen Fadenlänge L den längenbezogenen Mittelwert R als Qualitätsparameter

$$R = \frac{\overline{X}_{korr}}{L} \qquad , \qquad (II)$$

der einen objektiven Maßstab für die Beurteilung der Fadenqualität darstellt.

Findet sich in einem Wertekollektiv $X_1...X_n$ kein Ausreißerwert $\overset{*}{X}j$ wird $\overline{X}$ aus allen diesen Werten berechnet und daraus nach

$$R = \frac{\overline{X}}{L} \qquad (IIa)$$

der Wert von R berechnet. $\overline{X}$ ergibt sich selbstverständlich auch aus der Formel I mit N = Null.

Auch $\overline{X}$ bzw. $\overline{X}_{korr}$ kann als Qualitätsparameter benutzt werden, wenn z.B. im Rahmen eines Betriebsablaufs stets die gleiche Fadenlänge L gemessen wird. In diesem Fall kann die Normierung auf die Längeneinheit, d.h. die Division von $\overline{X}$ bzw. $\overline{X}_{korr}$ durch L unterbleiben.

Je nach Fragestellung, Interessenlage und Meßvorrichtung kann der Begriff "Ausreißerwert" unterschiedlich festgelegt werden.

Ein Ausreißerwert kann dadurch identifiziert werden, daß sein Einzelabweichungsquadrat vom Mittelwert aller Abweichungsquadrate mehr als $x$ mal so groß ist wie der Mittelwert aller Einzelabweichungsquadrate.

$$(\Delta \overset{*}{X}_j)^2 \geq \kappa \cdot \frac{\sum\limits_{i=1}^{i=n} (\Delta X_i)^2}{n} \qquad (III)$$

wobei $\Delta X_i = X_i - \overline{X}$ bedeutet und $x$ bei z. B. 3 hintereinander geschalteten Flusensensoren zweckmäßigerweise im Bereich von 1,78 bis 1,85, vorzugsweise 1,8 bis 1,83 liegt, und

$$\overline{X} = \frac{\sum\limits_{i=1}^{i=n} X_i}{n}$$

ist.

Ein abweichender Einzelwert wird umso eher als Ausreißerwert indiziert, je kleiner $x$ gewählt wird.

Als Ausreißerwert kann z.B. auch eine solcher Wert $X_j^*$ angesehen werden, dessen Einzelabweichungsquadrat vom Mittelwert $\overline{X}$ aller Meßwerte $X_i$ mehr als $\lambda$ mal so groß ist wie der Mittelwert aus den Einzelabweichungsquadraten aller Meßwerte $X_i$ mit Ausnahme von $X_j^*$ :

$$(\Delta X_j^*)^2 \geq \lambda \cdot \frac{\sum\limits_{i=1}^{i=n} (\Delta X_i)^2 - (\Delta X_j^*)^2}{n-1} \qquad (IV)$$

wobei $\Delta X_i$ bzw. $\Delta X_j^*$ die oben angegebene Bedeutung hat und $\lambda$ im Bereich von 2,7 bis 3,5, vorzugsweise von 3 bis 3,2 gewählt wird.

Erwartet man nur einen einzigen Ausreißerwert, was z. B. bei der Verwendung von drei Sensoren stets der Fall ist, so bewährt sich folgendes Vorgehen: Man errechnet, zunächst für jede mögliche Paarung ohne Wiederholung der Impulssummen $X_i$, nämlich $X_1/X_2$, $X_1/X_3$..., $X_1/X_n$; $X_2/X_3 .. X_2/X_n$ usw. die Beträge der Differenzen $D_{i1,i2}$

$$| X_{i1}-X_{i2} | = D_{i1,i2} \qquad (VII)$$

Wie man der Gleichung VII entnimmt, zeigen die Indices i1 und i2 an, daß der betreffende D-Wert aus den Impulssummenwerten $X_{i1}$ und $X_{i2}$ hervorgegangen ist. Entsprechend zeigen die Indices der weiter unter genannten Größen Y und Q an, aus welchen Paarungen von X-Werten sie hervorgegangen sind.

Bei n hintereinanderliegenden Flusensensoren ergeben sich hierbei

$$M = \sum\limits_{r=1}^{r=n-1} r \qquad (r= 1,2...n-1)$$

Werte für $D_{i1,i2}$; bei 3 Sensoren sind es demnach 3, bei 4 Sensoren 6 und bei 5 Sensoren 10 Differenzbeträge $D_{i1,i2}$.

Sodann wird der Zahlenwert von etwa 1, vorzugsweise 1 selbst,

a) entweder zu allen Werten $D_{i1,i2}$ oder

b) nur zu solchen Werten $D_{i1,i2}$, die Null sind,

hinzuaddiert und aus der so erhaltenen neuen Gruppe von Werten $Y_{i1,i2}$ der Minimalwert $Y^m_{i1,i2}$ herausgesucht. Danach bildet man die Quotienten

$$Q_{i1,i2} = \frac{Y_{i1,i2}}{Y^m_{i1,i2}}$$

und vergleicht diese mit einem für die Definition von Ausreißerergebnissen geeigneten Grenzwert G, der im Bereich von 2 bis 4, vorzugsweise bei 3 liegt.

Überschreitet keiner der erhaltenen $Q_{i1,i2}$-Werte diesen Grenzwert, so enthält das $X_i$-Kollektiv keinen Ausreißer und es wird aus allen Werten $X_i$ wie oben beschrieben der Mittelwert X, und gewünschtenfalls daraus der Qualitätsparameter R bestimmt.

Je nach den im Rechner gespeicherten Unterprogrammen kann es vorteilhaft sein, nicht alle möglichen $Q_{i1,i2}$-Werte zu berechnen, sondern zunächst sowohl den Minimalwert

$$Y^m_{i1,i2}$$

als auch den Maximalwert

$$Y^{max}_{i1,i2}\text{-Wert,}$$

und den Quotienten

$$Q_{max} = \frac{Y^{max}_{i1,i2}}{Y^m_{i1,i2}}$$

zu ermitteln. Man erhält so nur den größten der möglichen Quotienten $Q_{i1,i2}$. Ist er gleich oder kleiner als G, so enthält das $X_i$-Kollektiv keinen Ausreißer.

Der ausgewiesene Ausreißerwert von $X_1$ wird identifiziert und eliminiert, und aus dem verbleibenden Rest der Werte $X_i$ wie oben beschrieben der Mittelwert $\overline{X}^*$ und daraus R berechnet.

Zur Identifizierung des Ausreißerwertes $X^*_j$ werden die Indices der überhöhten $Q_{i1,i2}$-Werte herangezogen. In einem Kollektiv von 3 $X_i$-Werten erhält man 2, bei z.B. 5 $X_i$-Werten erhält man 4 überhöhte $Q^*_{i1,i2}$-Werte.

Der Ausreißerwert ist dabei derjenige Impulssummenwert $X_i$,dessen Index i in allen überhöhten $Q^*_{i1,i2}$-Werten wiederkehrt.

Hat sich das Vorhandensein eines Ausreißerwertes aus einem Vergleich von $Q_{max}$ und G ergeben, so wird zur Identifizierung des Ausreißerwerts $X^*_j$ die Indizierung des minimalen Y-Wertes

$$(Y^m_{i1,i2})$$

herangezogen: Der Ausreißerwert ist derjenige, dessen Index in

$$Y^m_{i1,i2}$$

nicht vorkommt:

$j \neq i1, j \neq i2$.

Zweckmäßigerweise werden 3 bis 5, vorzugsweise 3, Flusensensoren hintereinander in den Lauf des zu bewertenden Fadens eingeschaltet.

Überraschenderweise ergibt nämlich bereits der Einsatz von drei Sensoren je Fadenlauf gut reproduzierbare, aussagekräftige Messungen, sodaß meist auf den Aufwand mehrerer Sensoren verzichtet werden kann. Dieses einfache Verfahren mit nur drei Sensoren ist umso überraschender, als im Falle eines Ausreißers dort nur die Ergebnisse von zwei Sensoren berücksichtigt werden.

Ein weiteres einfaches Praxisverfahren besteht beim bevorzugten Einsatz von nur drei Sensoren darin, daß man ähnlich wie bereits geschildert, die drei Differenzen der drei Meßwerte $X_i$ bildet, Nullwerte durch Addition von vorzugsweise der Zahl 1 zu einem endlichen Wert macht und daraufhin die drei Quotienten Q bildet.

Leigt nun der größte Wert dieser drei Quotienten unter der vorgegebenen Grenze G, so werden alle drei Werte $X_i$ zum Wert $\overline{X}$ gemittelt, im anderen Fall werden nur die beiden Werte $X_{i1}$ und $X_{i2}$ benutzt, die den kleinsten Wert für die Differenz $D_{i1,i2} = |X_{i1} - X_{i2}|$ bilden.

Dieses Praxisverfahren zeichnet sich durch eine besonders hohe Prüfgeschwindigkeit aus. Es hat daher besondere Bedeutung für die Installation eines Echtzeitsystems.

Sehr gute Ergebnisse werden erhalten, wenn als Flusensensoren Lichtschranken eingesetzt werden. Diese können prinzipiell alle in gleicher Position bezüglich der Fadenlaufrichtig montiert werden. Es ist jedoch vorteilhaft, wenn die Sensoren hintereinander, jedoch in der Ebene senkrecht zur Fadenlaufrichtung um einen konstanten Winkel verdreht angeordnet sind.

Eine besonders gute Stabilisierung des Fadenlaufes und wenige Ausreißermeßwerte werden erhalten, wenn die Flusensensoren auf einem Bogen, bevorzugt einem Kreisbogen angeordnet sind, auf dem die Fäden zwangsgeführt werden.

Eine besondere Ausführungsart des erfindungsgemäßen Verfahrens besteht darin, daß als Flusensensoren Lichtschranken mit nachgeschalteter Klassifizierungselektronik eingesetzt werden, welche die Flusenzahlen auch noch nach der Flusengröße sortieren, wobei auch zwischen Quer- und Längserstreckung der Flusen unterschieden werden kann, und daß die so erhaltenen klassifizierten Impulse der Flusensensoren klassenweise ausgewertet werden.

Weitere zusätzliche Information über die Fadenqualität können nach dem Verfahren erhalten werden, wenn die Ereigniserfassung abschnittsweise erfolgt, d.h. in auf die gewählte Prüflänge bezogenen Fadenabschnittslängen, so daß Fehlerhäufungen bzw. periodisch auftretende Fehler erkannt und bestimmten Ursachen zugeordnet werden können.

Eine Vorrichtung zur Durchführung des erfindungsgemäßen Verfahrens besteht aus den mindestens drei im Fadenlauf hintereinander liegenden Flusensensoren, ggf. einer Einrichtung zur Messung der durchlaufenden Fadenlänge L, sofern man diese nicht bereits abgemessen vorlegt, und eine Recheneinheit, die die Zählimpulse für jeden Sensor extra zu den Impulssummen $X_i$ summiert, und diese Impulssummenwerte speichert, die erhaltenen Summen nach den oben angegebenen verfahrensspezifischen Kriterien auf Ausreißerwerte prüft und solche eliminiert, und sodann den Mittelwert aller zu berücksichtigenden Impulssummenwerte $X_i$ bildet und gewünschtenfalls diesen Mittelwert noch auf eine Fadenlängeneinheit normiert.

Als Einrichtung zur Messung der Fadenlänge kann jede hierfür bekannte Anordnung eingesetzt werden, z.B. eine vom laufenden Faden schlupffrei gedrehte Meßrolle bestimmten Umfangs, deren Umdrehungszahl registriert wird.

Eine solche erfindungsgemäße Vorrichtung kann noch ergänzt werden durch je ein vor und hinter der Gruppe der Flusensensoren im Fadenlauf liegendes unabhängig und stufenlos regelbares Lieferwerk, wobei die beiden Lieferwerke so geregelt werden, daß für verschiedene Garnarten die richtige Prüffadenspannung eingestellt und beibehalten wird. Das Beschleunigen der Fadenschar bis zur Prüfgeschwindigkeit bzw. das abschließende Abbremsen nach durchgeführter Prüfung erfolgt vorzugsweise über eine elektronische Steuerung, so daß keine Einfädelarbeiten erforderlich werden.

Die Recheneinheit der erfindungsgemäßen Vorrichtung prüft die gespeicherten Impulssummenwerte $X_i$ auf Ausreißerwerte, indem zunächst durch Mittelwertbildung aller auf die gewählte Fadenlänge L entfallenden Impulssummen die Hilfsrechengröße X gibildet wird und danach eine Rechenprogramm abläuft, das die einzelnen $X_i$-Werte daraufhin überprüft, ob sie einer der oben angegebenen Formeln III oder IV genügen. Selbstverständlich ist diese Prüfung alternativ, d.h. die Prüfung braucht nur wahlweise an einer der genannten Formeln zu erfolgen. Ein $X_i$-Wert, der einer der Formeln genügt, ist ein Ausreißerwert und wird vom Rechner aus dem Speicher gelöscht.

Ein alternatives Rechenprogramm zu Identifizierung von Ausreißerwerten bestimmt zunächst für jede mögliche Paarung der Impulssummenwerte $X_i$ die Differenzbeträge, addiert sodann

    a) entweder zu allen erhaltenen Differenzbeträgen oder

    b) nur zu Differenzbeträgen, die Null sind, einen Zahlenwert von etwa 1, vorzugsweise 1 selbst und speichert die erhaltenen Zwischenwerte $Y_{i1,i2}$ wobei die Indices als Identifikationsmerkmal mit abgespeichert werden.

Aus der erhaltenen Gruppe von Y-Werten sucht der Rechner nun den Minimalwert $Y^m_{i1,i2}$ und bildet für alle übrigen Y-Werte den Quotienten

$$Q_{i1,i2} = \frac{Y_{i1,i2}}{Y^m_{i1,i2}}$$

Der nächste Programmschritt besteht in einem Vergleich der Q-Werte mit einem gespeicherten Grenzwert G, einer Selektierung aller Q*-Werte, die diesen Grenzwert übersteigen, und der Bestimmung desjenigen Index, der in allen selektierten Q-*Werten vorkommt. Der so erhaltene Index ist der Index derjenigen Impulssumme $X_i$, die als Ausreißerwert zu betrachten ist.

Demgemäß identifiziert und behandelt das Rechenprogramm aus der Gruppe der gespeicherten $X_i$-Werte denjenigen als Ausreißerwert, der den in allen selektierten Q-werten vorkommenden Index aufweist.

Eine alternative Auswertung der erhaltenen $Y_{i1,i2}$-Werte besteht darin, daß der Rechner nicht nur den Minimalwert

$$Y^m_{i1,i2},$$

sondern auch den Maximalwert

$$Y^{max}_{i1,i2}$$

sucht und den Quotienten

$$Q_{max} = \frac{Y^{max}_{i1,i2}}{Y^m_{i1,i2}}$$

Ist $Q_{max}$ kleiner als G, so enthält das Kollektiv $X_i$ keinen Ausreißer, ist $Q_{max}$ größer als G, so enthält es einen Ausreißer. Dieser hat denjenigen Index, der in

$$Y^m_{i1,i2}$$

nicht vorkommt.

Die rechnerische Behandlung der Ausreißerwerte ist darauf gerichtet, diese Werte in geeigneter Weise aus der Mittelwertbildung zu eliminieren. Dies erfolgt durch Mittelwertbildung gemäß der Formel

$$\bar{X}^* = \frac{\sum_{i=1}^{i=n} X_i - X_i^*}{n-1} .$$

Die Recheneinheit kann hierzu z.B. auch so arbeiten, daß zunächst

$$\overline{X} = \frac{\displaystyle\sum_{i=1}^{i=n} X_i}{n}$$

gebildet wird und
anschließend

$$\overline{X}^* = \frac{n \cdot \overline{X} - X_i}{n-1}$$

errechnet wird
oder indem der Ausreißerwert $X_i^*$ erst gar nicht in die Mittelwertbildung einbezogen sondern aus dem Speicher der $X_i$-Werte eliminert wird. Anschließend errechnet der Rechner aus dem verbleibenden Rest der $X_i$-Werte den Mittelwert $\overline{X}^*$ und dividiert diesen gewünschtenfalls durch die Länge L des gemessenen Fadenabschnitts.

Unabhängig von der Rechenstrategie erhält man den durch die Formel (I) definierten Wert von $\overline{X}^*$.

Zur Durchführung des erfindungsgemäßen Verfahrens eignet sich besonders eine Anordnung, bei der jedes Garn einer Kettfadenschar zwei Lieferwerke und dazwischen mit definierter Garnspannung die mindestens drei hintereinander liegenden Lichtschranken durchläuft.

Aus der erfindungsgemäß bestimmten Flusenzahl pro Längeneinheit, eventuell aufgeschlüsselt nach vorgegebenen Größenklassen, lassen sich Qualitätskenndaten festlegen, die anwendungsbezogen den vagen Begriff "Garnqualität" objektivieren.

Diese Qualitätkenndaten können an einer Meßvorrichtung durch Wahl des Datenverarbeitungsprogramms dem jeweiligen Garn (Stapelfasergarn oder Multifilgarn), dem Titer (Einzel- und Gesamttiter), der Art des Garns (glatt, texturiert oder hochgedreht) und dem Einsatzgebiet angepaßt werden.

Der Vorteil des erfindungsgemäßen Verfahrens liegt nicht nur in der einfachen Anpassung an die Aufgabenstellung, d.h. an die Art der verwendeten Garne und die Qualitätsanforderungen, sondern das erfindungsgemäße Verfahren ersetzt darüber hinaus eine anstrengende, subjektiv beeinflußte, visuelle Überwachung am Schärgatter durch ein objektives, weitgehend automatisiertes Meßverfahren, das schnell viel differenziertere Aussagen ergibt.

Diese objektiven Qualitätsaussagen können in Form eines Prüfprotokolls oder einer einfachen Ja/Nein-Aussage zur Qualität erfolgen oder sie können gegebenenfalls sogar on line, zur Steuerung vorhergehender oder nachfolgender Produktionsprozesse verwendet werden.

Die nachfolgenden Ausführungsbeispiele veranschaulichen das erfindungsgemäße Verfahren.

Beispiel 1

Die erfindungsgemäße Prüfvorrichtung bestand aus drei hintereinander liegenden Lichtschranken, die auf einem Kreisbogen mit einem Krümmungsradius von 0,5 m angebracht waren. Die Strahlrichtung der Lichtschranken lagen in einer auf dem Fadenlauf senkrecht stehenden Ebene. Die gesamte Lichtschrankenanordnung lag zwischen zwei separat regelbaren Lieferwerken, die während des Versuchslaufs eine konstante Fadenspannung von 5 cN aufrechterhielten.

Den Lichtschranken war eine Recheneinheit nachgeschaltet, die die Lichtschrankenimpulse registrierte, summierte und speicherte. In einem weiteren Schritt wurden die Impulssummen der einzelnen Zähler gemäß einem der oben beschriebenen Verfahren weiterverarbeitet.

Die Fadengeschwindigkeit wurde auf 10 m/sec. eingestellt und der Fadenlauf gestartet. Nach einer Vorlaufzeit von 15 Sekunden, innerhalb der sich ein ruhiger und konstanter Fadenlauf einstellte, wurde die Messung gestartet und eine Fadenlänge von 3000 m ausgemessen.

Von den drei Lichtschranken wurden hierbei folgende Impulssummen erhalten:

$S_1 : X_1 = 14$; $S_2 : X_2 = 3$; $S_3 : X_3 = 3$.

Die Weiterbehandlung dieser Werte im Rechner ergab folgende Zwischenergebnisse

$D_{1,2} = |X_1 - X_2| = 11$
$D_{1,3} = |X_1 - X_3| = 11$
$D_{2,3} = |X_2 - X_3| = 0$

Zu den Wert $D_{2,3}$, dessen Betrag 0 ist wurde 1 addiert und so $Y_{1,2} = 11$; $Y_{1,3} = 11$; $Y_{2,3} = 1$ erhalten.
Selektion des kleinsten Y-Wertes und Division der übrigen Y-Werte durch den kleinsten ergab:

$$Q^*_{1,2} = \frac{Y_{1,2}}{Y_{2,3}} = \frac{11}{1} = 11$$

und analog
$Q^*_{1,3} = 11$ und $Q_{2,3} = 1$. Der dem Rechner eingegebene Grenzwert G war 3 und demnach wurden $Q^*_{1,2}$ und $Q^*_{1,3}$ mit jeweils 11 als weit überhöht bewertet.
Der den beiden überhöhten Q*-Werten gemeinsame Index wurde vom Rechner als 1 erkannt und somit $X_1$ als Ausreißerwert identifiziert und eliminert.
Der schließlich aus $X_2$ und $X_3$ gebildete Mittelwert $\overline{X}^*$ wurde zu 3 ermittelt und ausgegeben.
Zu dem gleichen Ergebnis gelangte man durch ein alternatives Rechenprogramm:
Selektion des größten und kleinsten Y-Wertes und Division des größten Y-Wertes durch den kleinsten ergab:

$$Q_{max} = \frac{Y_{1,2}}{Y_{2,3}} = \frac{11}{1} = 11$$
$$*$$

Da der dem Rechner eingegebene Grenzwert G für den $Q_{max}$, aus $Y_{max}$ und $Y_{min}$ 3 war, erkannte er bei einem Vergleich mit dem Ergebnis $Q_{max} = 11$ das Vorhandensein eines Ausreißerwertes.
Als diesen Ausreißerwert identifizierte er den nicht an der kleinsten Y-Differenz beteiligten Sensor $S_1$ mit $X_1 = 14$.
Bei 10 Wiederholungen der Messung an der gleichen Garnqualität lagen alle Meßergebnisse $\overline{X}$ zwischen 2 und 5. Das Verfahren ist somit sehr gut reproduzierbar.
Bei der Messung einer gleichen Garnqualität nach herkömmlicher Methode mit einer Lichtschranke wurde bei 10 Messungen 6 mal $\overline{X}$-Werte zwischen 2 und 10 und 4 mal $\overline{X}$-Werte zwischen 10 und 20 erhalten. Die bekannte Methode ist somit nicht zur einwandfreien Bewertung der Garnqualität geeignet.
Für die Produktionsprüfung wird ein Prüfgerät mit 20 nebeneinanderliegenden Fadenlaufpositionen à 3 hintereinander angeordneter Lichtschranken angefertigt.

Beispiel 2

Eine Meßvorrichtung wie in Beispiel 1 beschrieben jedoch mit 4 Lichtschranken wurde zur Messung eines Garns eingesetzt.
Bei einem Meßlauf, der ein Ausreißerergebnis zeigte, wurden folgende Daten ermittelt und über die angegebenen Zwischenwerte zu dem $\overline{X}$-Wert verarbeitet:
$X_1 = 4$; $X_2 = 5$; $X_3 = 13$; $X_4 = 3$
$D_{1,2} = 1$; $D_{1,3} = 9$; $D_{1,4} = 1$; $D_{2,3} = 8$; $D_{2,4} = 2$; $D_{3,4} = 10$
$Y_{1,2} = 1$; $Y_{1,3} = 9$; $Y_{1,4} = 1$; $Y_{2,3} = 8$; $Y_{2,4} = 2$; $Y_{3,4} = 10$
von den kleinsten Y-Werten verwendete der Rechner den mit der kleinsten Index-Zahl : $Y_{1,2}$
$Q_{1,2} = 1$; $Q_{1,3} = 9$; $Q_{1,4} = 1$; $Q_{2,3} = 8$; $Q_{2,4} = 2$; $Q_{3,4} = 10$
Q-Werte > 2 sind $Q^*_{1,3}$; $Q^*_{2,3}$; $Q^*_{3,4}$ (Ausreißerwerte)
gemeinsamer Index: 3;
Ausreißerwert der Impulssumme = $X_3$.

9

Mittelwertbildung daher aus $X_1$, $X_2$ und $X_4$

Ergebnis $\overline{X}$ = 4.

Bei 10 Wiederholungen der Messung an der gleichen Garnqualität wurden 6 mal Ergebnisse zwischen 3 und 5 und 1 mal das Ergebnis 6 erhalten. Die Reproduzierbarkeit der Messung ist somit für die automatische Überwachung von Schergattern völlig ausreichend. Ein Einzelzähler zeigte dagegen bei 10 Messungen 3 mal Flusenzahlen über 30 an.

Beispiel 3

Eine Meßvorrichtung gemäß Beispiel 1, jedoch mit einer den drei Flusensensoren nachgeschalteten Bewertungsschaltungen für große mittlere und kleine Flusen sowie für Flusennester wurde zur Messung eines Garns eingesetzt.

Folgende Einzelergebnisse wurden von den Sensoren angezeigt

| Flusengröße | $X_1$ | $X_2$ | $X_3$ |
|---|---|---|---|
| Groß | 1 | 0 | 0 |
| Mittel | 3 | 1 | 0 |
| klein | 11 | 2 | 3 |
| Nest | 0 | 0 | 0 |

Die klassenweise Auswertung der $X_i$-Werte durch den Rechner nach dem in Beispielen 1 und 2 angegebenen Verfahren ergab bei einem dem Rechner eingegebenen Grenzwert G von 3:

Große Flusen

$D_{1,2} = 1\ Y_{1,2} = 1\ Q_{1,2} = 1$
$D_{1,3} = 1\ Y_{1,3} = 1\ Q_{1,3} = 1$
$D_{2,3} = 0\ Y_{2,3} = 1\ Q_{2,3} = 1$

Mittlere Flusen

$D_{1,2} = 2\ Y_{1,2} = 2\ Q_{1,2} = 2$
$D_{1,3} = 3\ Y_{1,3} = 3\ Q_{1,3} = 3$
$D_{2,3} = 1\ Y_{2,3} = 1\ Q_{2,3} = 1$

Kleine Flusen

$D_{1,2} = 9\ Y_{1,2} = 9\ Q^*_{1,2} = 9$
$D_{1,3} = 8\ Y_{1,3} = 8\ Q^*_{1,3} = 8$
$D_{2,3} = 1\ Y_{2,3} = 1\ Q_{2,3} = 1$
Demgemäß ergeben sich folgende Ergebnisse
$\overline{X}$ f. große Flusen : 0,3
$\overline{X}$ f. mittlere Flusen : 1.3
$\overline{X}$ f. kleine Flusen : 2,5
Bei allen in den Beispielen angegebenen Messungen standen die Meßergebnisse unmittelbar nach Durchlauf der Meßlänge des Garns zur Verfügung.

Beispiel 4

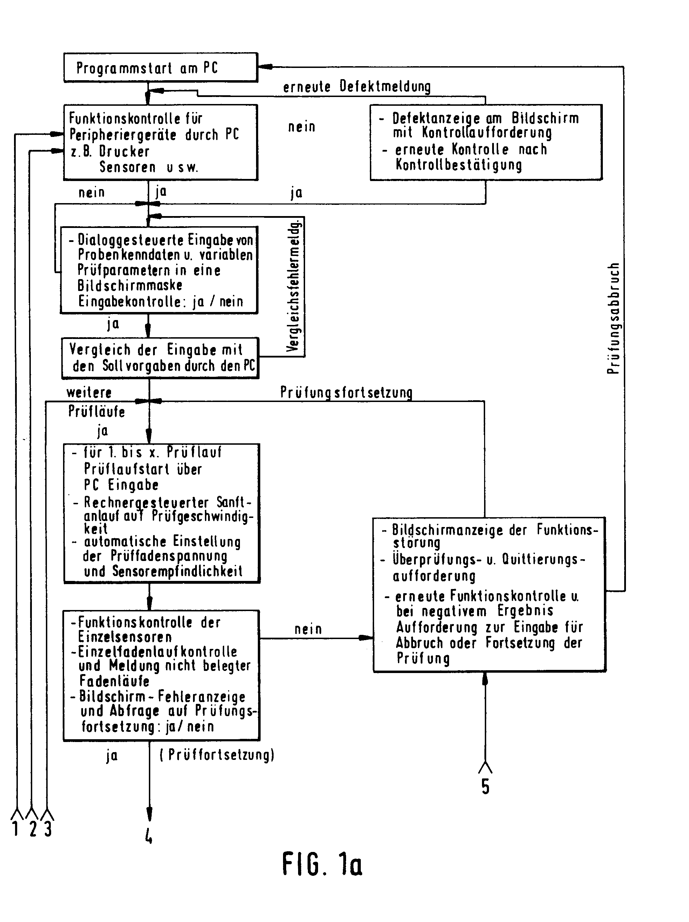

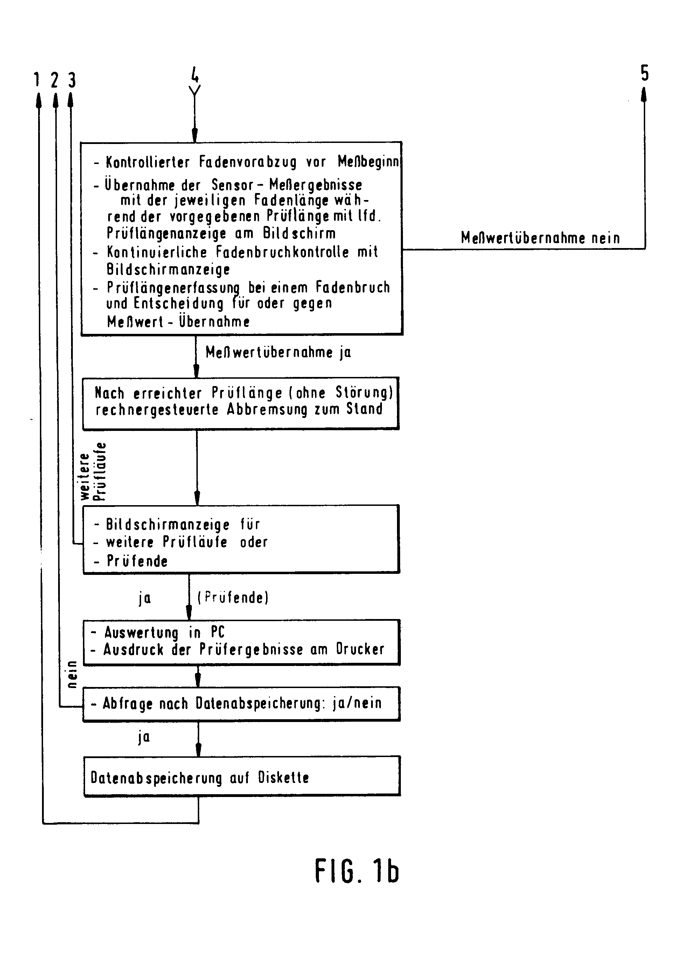

Das auf den Figuren 1a und 1b wiedergegebene Flußdiagramm veranschaulicht eine mögliche, praxisbezogene Ausgestaltung des erfindungsgemäßen Verfahrens und der dazu eingesetzten Meßvorrichtung.

**Patentansprüche**

**1.** Verfahren zur Bestimmung von Qualitätskenndaten an laufenden Garnen, bei dem die Zahl der Garnstörungen mit Hilfe von Flusenzählern ermittelt wird, dadurch gekennzeichnet, daß der Faden mindestens drei gleichartige, hintereinander angeordnete Flusensensoren durchläuft, deren bei Durchlauf eines Garnabschnitts der frei gewählten Länge L abgegebenen Zählimpulse in einen längenbezogenen Mittelwert R umgerechnet werden, wobei weit abweichende Einzelmeßergebnisse als Ausreißerwerte nicht in die Mittelwertbildung einbezogen werden.

2. Verfahren nach Anspruch 1 , dadurch gekennzeichnet, daß in Fadenlaufrichtung drei bis n Flusensensoren $S_1$ bis $S_n$ hintereinander angeordnet sind, die während des Durchlaufs eines Fadenabschnitts der frei wählbaren, nicht zu kurzen Länge L von den einzelnen Sensoren abgegebenen Zählimpulse für jeden der Flusensensoren $S_1$ bis $S_n$ getrennt zu den Impulssummen $X_1$ bis $X_n$ addiert und gespeichert werden, eventuell vorhandene Werte $X_j^*$ , die stark von den übigen Werten $X_i$ abweichen, als Ausreißerwert identifiziert und eliminiert werden, indem der Mittelwert $\overline{X}^*$ nach der Formel

$$\overline{X}^* = \frac{\sum_{i=1}^{i=n} X_i - \sum_{j=1}^{j=N} X_j^*}{n-N}$$

berechnet
und ggf. hieraus durch Normierung auf eine Längeneinheit der gemessenen Fadenlänge L den längebezogenen Mittelwert R als Qualitätsparameter

$$R = \frac{\overline{X}^*}{L}$$

berechnet wird.

3. Verfahren nach mindestens einem der Ansprüche 1 und 2, dadurch gekennzeichnet, daß der Ausreißerwert $X_i^*$ dadurch identifiziert wird, daß er eine Einzelabweichung $\Delta X_i^*$ vom Mittelwert der Einzelabweichungen aufweist, die alternativ einer der folgenden Formeln genügt:

$$(\Delta X_i^*)^2 \geq \kappa \cdot \frac{\sum_{i=1}^{i=n} (\Delta X_1)^2}{n}$$

oder

$$(\Delta X_i^*)^2 \geqslant \lambda \cdot \frac{\sum_{i=1}^{i=n} (\Delta X_i)^2 - (\Delta X_i^*)^2}{n-1} \quad ,$$

wobei $\Delta X_i = \overline{X}_i - X$

11

$$\overline{X} = \frac{\sum\limits_{i=1}^{i=n} X_i}{n}$$

ist und

$x$ eine Zahl von 1,78 bis 1,85 und

$\lambda$ eine Zahl von 2,7 bis 3,5 bedeutet.

**4.** Verfahren nach mindestens einem der Ansprüche 1 und 2, dadurch gekennzeichnet, daß die Identifizierung der Ausreißerwerte erfolgt indem man zunächst für jede mögliche Paarung ohne Wiederholung der Impulssummen $X_i$, die Beträge der Differenzen $D_{i1,i2}$ errechnet

$| X_{i1} - X_{i2} | = D_{i1,i2},$

sodann einen Zahlenwert von etwa 1, vorzugsweise 1 selbst,

    a) entweder zu allen Werten $D_{i1,i2}$ oder

    b) nur zu solchen Werten $D_{i1,i2}$, die Null sind,

hinzuaddiert, und aus der so erhaltenen neuen Gruppen von Werten $Y_{i1,i2}$ der Minimalwert $Y^m_{i1,i2}$ heraussucht, danach die Quotienten

$$Q_{i1,i2} = \frac{Y_{i1,i2}}{Y^m_{i1,i2}}$$

berechnet,

alle diejenigen $Q^*_{i1,i2}$-Werte feststellt, die einen Grenzwert G überschreiten und schließlich dem Kollektiv der $X_i$-Werte denjenigen als Ausreißerwert entnimmt, dessen Index j in allen $Q^*_{i1,i2}$-Werten auftaucht.

**5.** Verfahren nach mindestens einem der Ansprüche 1 und 2, dadurch gekennzeichnet, daß drei Flusensensoren eingesetzt werden und die Identifizierung der Ausreißerwerte erfolgt, indem man zunächst für jede mögliche Paarung ohne Wiederholung der Impulssummen $X_i$, die Beiträge der Differenzen $D_{i1,i2}$ errechnet

$| X_{i1} - X_{i2} | = D_{i1,i2},$

sodann einen Zahlenwert von etwa 1, vorzugsweise 1 selbst,

    a) entweder zu allen Werten $D_{i1,i2}$ oder

    b) nur zu solchen Werten $D_{i1,i2}$, die Null sind,

hinzuaddiert, und aus der so erhaltenen neuen Gruppe von Werten $Y_{i1,i2}$ den Minimalwert $Y^m_{i1,i2}$ und den Maximalwert $Y^{max}_{i1,i2}$ heraussucht, danach den Quotienten

$$Q_{max} = \frac{Y^{max}_{i1,i2}}{Y^m_{i1,i2}}$$

berechnet,

diesen Wert mit einem Grenzwert G vergleicht und falls $Q_{max} > G$ dem Kollektiv der $X_i$-Werte denjenigen als Ausreißerwert entnimmt, dessen Index j im Index von

$$Y^m_{i1,i2}$$

nicht enthalten ist.

6. Verfahren nach Anspruch 5, dadurch gekennzeichnet, daß der größte der drei Quotienten $Q_{i1,i2}$ mit G verglichen wird und,
falls er kleiner ist als G, alle drei $X_i$-Werte zum Wert X gemittelt,
falls er größer ist als G, zur Berechnung von $\overline{X}$ nur die beiden $X_i$-Werte benutzt werden, die den kleinsten Differenzbetrag

$$D_{i1,i2} = |\,X_{i1} - X_{i2}\,|$$

bilden.

7. Verfahren nach mindestens einem der Ansprüche 1 bis 6, dadurch gekennzeichnet, daß als Flusensensor eine Lichtschranke eingesetzt wird.

8. Verfahren nach mindestens einem der Ansprüche 1 bis 6, dadurch gekennzeichnet, daß Flusensensor eine Lichtschranke mit nachgeschalteter Klassifizierungselektronik eingesetzt wird.

9. Verfahren nach Anspruch 8, dadurch gekennzeichnet, daß die klassifizierten Impulse der Flusensensoren klassenweise ausgewertet werden.

10. Verfahren nach mindestens einem der Ansprüche 1 bis 9, dadurch gekennzeichnet, daß 3 bis 5 Flusensensoren hintereinander im Fadenlauf liegen.

11. Vorrichtung zur Bestimmung objektiver Qualitätsparameter eines Fadens bzw. Garns gekennzeichnet durch folgende Anordnung: mindestens drei in Fadenlauf hintereinander liegenden Flusensensoren, ggf. einer Einheit zur Messung der durchlaufenden Fadenlänge L und einer Recheneinheit, die die Zählimpulse für jeden Sensor extra zu den Impulssummenwerten $X_i$ summiert und diese Werte speichert, die Gruppe der erhaltenen $X_i$-Werte auf Ausreißerwerte $X_i^*$ prüft und diese dadurch eliminert, daß sie einen Mittelwert $\overline{X}^*$ gemäß der Formel

$$\overline{X}^* = \frac{\displaystyle\sum_{i=1}^{i=n} X_i - \sum_{j=1}^{j=N} X_j^*}{n-N}$$

ermittelt und diesen gewünschtenfalls auf die Fadenlängeneinheit normiert.

12. Vorrichtung gemäß Anspruch 11, ergänzt durch je ein vor und hinter der Gruppe der Flusensensoren im Fadenlauf liegendes; unabhängig und stufenlos regelbares Lieferwerk, wobei beide Lieferwerke so geregelt werden, daß für verschiedene Garnarten die richtige Prüfspannung eingestellt und beibehalten wird.

13. Vorrichtung gemäß Anspruch 11, ergänzt durch je ein vor und hinter der Gruppe der Flusensensoren im Fadenlauf liegendes, unabhängig und stufenlos regelbares Lieferwerk, dadurch gekennzeichnet, daß das Beschleunigen der Lieferwerke bis zur Prüfgeschwindigkeit bzw. das Abbremsen nach erfolgter Prüfung vorzugsweise über elektronische Steuerung so erfolgt, daß keine Einfädelarbeiten erforderlich werden.

## Claims

1. A method of determining the quality characteristics of running yarns, in which the number of yarn imperfections is ascertained with the aid of fluff counters, wherein the thread passes through at least three fluff sensors of the same type arranged one after another, whose counting pulses, which are emitted upon passage by a yarn section of the freely selectable length L, are converted into a length-related average R, highly deviant individual measurement results being regarded as outlier values and not being included in the averaging.

2. The method as claimed in claim 1, wherein three to n fluff sensors $S_1$ to $S_n$ are arranged one after another in the direction of the thread path, the counting pulses emitted by the individual sensors during the passage by a thread section of the freely selectable length L, which is not too short, are added separately to the pulse totals $X_1$ to $X_n$ for each of the fluff sensors $S_1$, to $S_n$, and are stored, and any values present of $X_j^*$ which deviate markedly from the remaining values of $X_i$ are identified and eliminated as outlier values, by calculating the average $\overline{X}^*$ with the formula

$$\overline{X}^* = \frac{\sum\limits_{i=1}^{i=n} X_i - \sum\limits_{j=1}^{j=N} X_j^*}{n-N}$$

and, if necessary, using this value to compute the length-related average R as quality parameter

$$R = \frac{\overline{X}^*}{L}$$

by scaling to a length unit of the measured thread length L.

3. The method as claimed in one or more of claims 1 and 2, wherein the outlier value $X_i^*$ is identified in that it exhibits an individual deviation $\Delta X_i^*$ from the average of the individual deviations, which alternatively satisfies one of the following formulae:

$$(\Delta X_i^*)^2 \geq \kappa \cdot \frac{\sum\limits_{i=1}^{i=n} (\Delta X_i)^2}{n}$$

or

$$(\Delta X_i^*)^2 \geqslant \lambda \cdot \frac{\sum\limits_{i=1}^{i=n} (\Delta X_i)^2 - (\Delta X_i^*)^2}{n-1}$$

14

where

$$\Delta X_i = \overline{X}_i - X$$

$$\overline{X} = \frac{\sum\limits_{i=1}^{i=n} X_i}{n}$$

$x$      denotes a number from 1.78 to 1.85 and

$\lambda$      denotes a number from 2.7 to 3.5.

4. The method as claimed in one or more of claims 1 and 2, wherein the identification of the outlier values takes place in the manner that, firstly, the values of the differences $D_{i1,i2}$

$$|X_{i1-xi2}| = D_{i1,i2}$$

are computed for each possible pairing without repetition of the pulse totals $X_i$, thereafter, a numerical value of about 1, preferably 1 itself, is further added
   a) either to all the values $D_{i1,i2}$ or
   b) only to such values $D_{i1,i2}$ as vanish, and the minimum value $Y^m_{i1,i2}$ is extracted from the new groups of values $Y_{i1,i2}$ so obtained,
   the quotients

$$Q_{i1,i2} = \frac{Y_{i1,i2}}{Y^m_{i1,i2}}$$

are then formed, all the values of $Q^*_{i1,i2}$ exceeding a limiting value G are determined and, finally, from the population of $X_i$ values there is chosen as outlier value the one whose index j occurs in all values of $Q^*_{i1,i2}$.

5. The method as claimed in one or more of claims 1 and 2, wherein three fluff sensors are employed, and the identification of the outlier values takes place in the manner that, firstly, the amounts of the differences $D_{i1,i2}$

$$|X_{i1}-X_{i2}| = D_{i1,i2}$$

are computed for each possible pairing without repetition of the pulse totals $X_i$, thereafter, a numerical value of about 1, preferably 1 itself, is further added
   a) either to all values $D_{i1,i2}$ or
   b) only to such values $D_{i1,i2}$ as vanish, and the minimum value $Y^m_{i1,i2}$ and the maximum value $Y^{max}_{i1,i2}$ are extracted from the new group of values $Y_{i1,i2}$ so obtained, the quotient

$$Q_{max} = \frac{Y^{max}_{i1,i2}}{Y^m_{i1,i2}}$$

15

is then computed, this value is compared with a limiting value G and, if $Q_{max} > G$, from the population of $X_i$ values there is chosen as outlier value the one whose index j is not contained in the index of

$$Y^m_{i1,i2}$$

6. The method as claimed in claim 5, wherein the largest of the three quotients $Q_{i1,i2}$ is compared with G and, if it is smaller than G, all three $X_i$ values are averaged for the value X, and, if it is larger than G, then in order to compute $\overline{X}$ only the two $X_i$ values are used which generate the smallest difference amount

$$D_{i1,i2} = | X_{i1} - X_{i2} |$$

7. The method as claimed in one or more of claims 1 to 6, wherein a light barrier is employed as fluff sensor.

8. The method as claimed in one or more of claims 1 to 6, wherein a light barrier with classification electronics connected downstream is employed as fluff sensor.

9. The method as claimed in claim 8, wherein the classified pulses of the fluff sensors are evaluated by classes.

10. The method as claimed in one or more of claims 1 to 9, wherein 3 to 5 fluff sensors are located one after another in the thread path.

11. A device for determining objective quality parameters of a thread or yarn, which comprises the following arrangement: at least three fluff sensors located one after another in the thread path, if necessary a device for measuring the length L of the thread passing through and an arithmetic unit which, in addition to the pulse total values $X_i$, sums the counting pulses for each sensor and stores these values, tests the group of the $X_i$ values obtained for outlier values $X_i'$ and eliminates the latter, by determining an average $\overline{X}^*$ according to the formula

$$\overline{X}^* = \frac{\sum_{i=1}^{i=n} X_i - \sum_{j=1}^{j=N} X_j^*}{n-N}$$

and scales the latter, if desired, to the thread length unit.

12. The device as claimed in claim 11, supplemented both before and after the group of fluff sensors by a feed system which is located in the thread path and may be controlled independently and in a stepless fashion, the two feed systems being controlled in such a way that for various yarn types the correct test tension is set and maintained.

13. The device as claimed in claim 11, supplemented both before and after the group of fluff sensors by a feed system which is located in the thread path and may be controlled independently and in a stepless fashion, wherein the acceleration of the feed systems up to the test speed or the braking after the completed test is preferably done via an electronic control in such a way that no threading-in is required.

**Revendications**

1. Procédé pour déterminer la qualité de fils élémentaires en défilement, selon lequel le nombre des défauts de fils est déterminé à l'aide de compteurs de peluches, caractérisé en ce que le fil passe par au moins trois capteurs de peluches similaires disposés l'un derrière l'autre, dont les impulsions de comptage générées lors du passage d'une section de fil de la longueur L librement choisie sont converties en une valeur de longueur moyenne R, les résultats de mesures individuelles qui présentent un écart très important étant éliminés comme valeurs aberrantes et n'étant pas intégrés dans le calcul de la valeur moyenne.

2. Procédé selon la revendication 1, caractérisé en ce que trois à n capteurs de peluches $S_1$ à $S_n$ sont disposés en série dans le sens de l'avance du fil, en ce que les impulsions de comptage générées par les différents capteurs pendant le passage d'une section de fils de la longueur L librement choisie, mais pas trop courte, sont additionnées aux sommes d'impulsions $X_1$ à $X_n$ et ce, séparément pour chaque capteur de peluches $S_1$ à $S_n$, puis mémorisées, et en ce que d'éventuelles valeurs $X^*_j$ s'écartant considérablement des autres valeurs $X_i$ sont identifiées comme valeurs aberrantes et éliminées, la valeur moyenne X* étant calculée selon la formule :

$$\overline{X}^* = \frac{\displaystyle\sum_{i=1}^{i=n} X_i - \sum_{j=1}^{j=N} X_j^*}{n-N}$$

et la valeur moyenne R étant éventuellement calculée, comme paramètre de qualité, sur la base de cette formule, par normalisation de la longueur de fils L mesurée par rapport à une unité de longueur :

$$R = \frac{X^*}{L}$$

3. Procédé selon la revendication 1 ou 2, caractérisé en ce que la valeur abérrante $X^*_i$ est identifiée par le fait qu'elle présente un écart individuel delta $X^*_i$ par rapport à la valeur moyenne des écarts individuels correspondant alternativement à l'une des deux formules suivantes :

$$(\Delta X_i^*)^2 \geq \kappa \cdot \frac{\displaystyle\sum_{i=1}^{i=n} (\Delta X_i)^2}{n}$$

ou

$$(\Delta X_i^*)^2 \geq \lambda \cdot \frac{\displaystyle\sum_{i=1}^{i=n} (\Delta X_i)^2 - (\Delta X_i^*)^2}{n-1}$$

avec :

EP 0 296 469 B1

$$\Delta X_i = \overline{X}_i - X$$

$$\overline{X} = \frac{\displaystyle\sum_{i=1}^{i=n} X_i}{n}$$

et

$x$ correspondant à une valeur comprise entre 1,78 et 1,85 et
$\lambda$ correspondant à une valeur comprise entre 2,7 et 3,5.

**4.** Procédé selon l'une quelconque des revendications 1 et 2, caractérisé en ce que l'identification des valeurs abérrantes est effectuée en calculant, pour chaque couple possible, sans répétition des sommes d'impulsions $X_i$, les sommes des différences des $D_{i1,i2}$ :

$$| X_{i1} - X_{i2} | = D_{i1,i2},$$

et en additionnant, par la suite, une valeur numérique d'approximativement 1, de préférence, de 1
   a) soit à l'ensemble des valeurs $D_{i1,i2}$
   b) soit Uniquement aux valeurs $D_{i1,i2}$ égales à zéro,
en choisissant, parmi les nouveaux groupes de valeurs $Y_{i1,i2}$ obtenus de cette manière, la valeur minimale $Y^m_{i1,i2}$ et en calculant, par la suite, les quotients :

$$Q_{i1,i2} = \frac{Y_{i1,i2}}{Y^m_{i1,i2}}$$

en définissant l'ensemble des valeurs $Q^*_{i1,i2}$ supérieures à une valeur limite G et en sélectionnant, dans le collectif des valeurs $X_i$, la valeur dont l'indice j se présente dans l'ensemble des valeurs $Q^*_{i1,i2}$ comme valeur défectueuse.

**5.** Procédé selon la revendications 1 ou 2, caractérisé en ce que trois capteurs de peluches sont utilisés et en ce que l'identification des valeurs défectueuses est effectuée en calculant, dans un premier temps, pour chaque couple possible, sans répétition des sommes d'impulsions $X_i$, les sommes des différences des $D_{i1,i2}$ :

$$| X_{i1} - X_{i2} | = D_{i1,i2},$$

et en additionnant, par la suite, une valeur numérique d'approximativement 1, de préférence, de 1,
   a) soit à l'ensemble des valeurs $D_{i1,i2}$
   b) soit uniquement aux valeurs $D_{i1,i2}$ égales à zéro,
en choisissant, parmi les nouveaux groupes de valeurs $Y_{i1,i2}$ obtenus de cette manière, la valeur minimale $Y^m_{i1,i2}$ et la valeur maximale $Y^{max}_{i1,i2}$ en calculant, par la suite, les quotients :

$$Q_{maxi} = \frac{Y^{maxi}_{i1,i2}}{Y^m_{i1,i2}}$$

en comparant cette valeur à une valeur limite G et en choisissant, pour $Q_{maxi} > G$, dans le collectif des

18

valeurs $X_i$, la valeur dont l'indice j n'est pas comprise comme valeur défectueuse dans l'indice de :

$Y^m_{i1,i2}$ .

**6.** Procédé selon la revendication 5, caractérisé en ce que le plus grand des trois quotients $Q_{i1,i2}$ est comparé avec G et en ce que, dans le cas où ce quotient est inférieur à G, toutes les trois valeurs $X_i$ sont utilisées pour calculer la valeur moyenne X ou, si le quotient est supérieur à G, seules les deux valeurs $X_i$ sont utilisées pour le calcul de X dont la différence :

$D_{i1,i2} = |X_{i1} - X_{i2}|$

fournit la valeur la plus petite.

**7.** Procédé selon l'une quelconque des revendications 1 à 6, caractérisé en ce que le capteur de peluches est une barrière lumineuse.

**8.** Procédé selon l'une quelconque des revendications 1 à 6, caractérisé en ce que le capteur de peluches utilisé est une barrière lumineuse reliée à une unité électronique de classification.

**9.** Procédé selon la revendication 8, caractérisé en ce que les impulsions classifiées des capteurs de peluches sont exploitées par classes.

**10.** Procédé selon l'une quelconque des revendications 1 à 9, caractérisé en ce que trois à cinq capteurs de peluches sont installés en série dans le passage du fil.

**11.** Dispositif pour déterminer la qualité de fils élémentaires en défilement, caractérisé par la disposition suivante : au moins trois capteurs de peluches montés en série dans le passage du fil, éventuellement une unité de mesure de la longueur L du fil et une unité de calcul qui additionne les impulsions de comptage de chaque capteur aux sommes des impulsions $X_i$ et qui mémorise ces valeurs, contrôle le groupe des valeurs $X_i$ obtenues pour sélectionner des valeurs défectueuses $X^*_i$, et élimine ces dernières, en ce qu'elle calcule une valeur moyenne $X^*$ selon la formule :

$$\overline{X}^* = \frac{\sum_{i=1}^{i=n} X_i - \sum_{j=1}^{j=N} X^*_j}{n-N}$$

et en ce qu'elle normalise, le cas échéant, cette valeur par rapport à l'unité de la longueur de fil.

**12.** Dispositif selon la revendication 11, équipé d'un dispositif complémentaire situé derrière le groupe de capteurs de peluches dans le passage du fil, chaque dispositif d'alimentation pouvant être réglé indépendamment et en continu, les deux dispositifs d'alimentation étant réglés de manière à assurer et maintenir la tension de contrôle appropriée pour chaque type de fil.

**13.** Dispositif selon la revendication 11, équipé d'un dispositif complémentaire situé avant le groupe des capteurs de peluches dans le passage du fil et par un dispositif d'alimentation situé derrière ce groupe, les deux dispositifs étant réglables indépendamment et en continu, caractérisé en ce que l'accélération des dispositifs d'alimentation à la vitesse de contrôle, respectivement leur décélération, après le contrôle est commandée, de préférence, par une commande électronique de manière à éviter toute nécessité d'effectuer des opérations d'enfilage.

EP 0 296 469 B1

FIG. 1a

20

1 2 3 4 5

- Kontrollierter Fadenvorabzug vor Meßbeginn
- Übernahme der Sensor - Meßergebnisse
    mit der jeweiligen Fadenlänge wäh-
    rend der vorgegebenen Prüflänge mit lfd.
    Prüflängenanzeige am Bildschirm
- Kontinuierliche Fadenbruchkontrolle mit
  Bildschirmanzeige
- Prüflängenerfassung bei einem Fadenbruch
  und Entscheidung für oder gegen
  Meßwert - Übernahme

Meßwertübernahme nein

Meßwertübernahme ja

Nach erreichter Prüflänge (ohne Störung)
rechnergesteuerte Abbremsung zum Stand

weitere Prüfläufe

- Bildschirmanzeige für
- weitere Prüfläufe oder
- Prüfende

ja   (Prüfende)

- Auswertung in PC
- Ausdruck der Prüfergebnisse am Drucker

nein

- Abfrage nach Datenabspeicherung: ja/nein

ja

Datenabspeicherung auf Diskette

# FIG. 1b

21